Europäisches Patentamt

European Patent Office

Office européen des brevets

(11) Publication number : **0 546 349 A2**

## (12) EUROPEAN PATENT APPLICATION

(21) Application number : 92119535.0

(22) Date of filing : 16.11.92

(51) Int. Cl.⁵ : **C12N 15/74,** C12N 1/21, C12N 15/65, C12P 19/04, // (C12N1/21, C12R1:21)

(30) Priority : 09.12.91 US 803866

(43) Date of publication of application : 16.06.93 Bulletin 93/24

(84) Designated Contracting States : AT BE CH DE DK ES FR GB GR IE IT LI LU NL PT SE

(71) Applicant : **AMERICAN CYANAMID COMPANY 1937 West Main Street P.O. Box 60 Stamford Connecticut 06904-0060 (US)**

(72) Inventor : **Finn, Charles William, Jr. 1 Johnson Road Andover, Massachusetts 01810 (US)**

(74) Representative : **Wächtershäuser, Günter, Dr. Tal 29 W-8000 München 2 (DE)**

(54) PRP overproducing haemophilus influenzae strain.

(57) The present invention provides novel strains of Haemophilus influenzae type b which produce large quantities of the capsular polysaccharide polyribosyl-ribitol-phosphate. Also provided is a method for producing such micro-organisms.

EP 0 546 349 A2

## FIELD OF THE INVENTION

The present invention relates to a recombinant microorganism useful in production of vaccine components. More specifically, the invention relates to a strain of Haemophilus influenzae type b which produces unusually large quantities of the capsular polymer polyribosyl-ribitol-phosphate (PRP)

## BACKGROUND OF THE INVENTION

Haemophilus influenzae b (Hib) is the causative agent of a type of pneumonia which is particularly dangerous to young children. A number of vaccines are currently available which are said to protect children of varying ages against Hib infection. An important component of certain vaccines is the polysaccharide polyribosyl-ribitol-phosphate, or PRP, a capsular polymer of Hib. Although PRP per se is a relatively poor immunogen, it has proven highly immunogenic in conjugated form. As a valuable vaccine component, there is substantial demand for PRP, which currently is only obtainable by isolation and purification from cultured. In the absence of a synthetic means for producing PRP, it would be desirable to have a high yield microbial source of PRP.

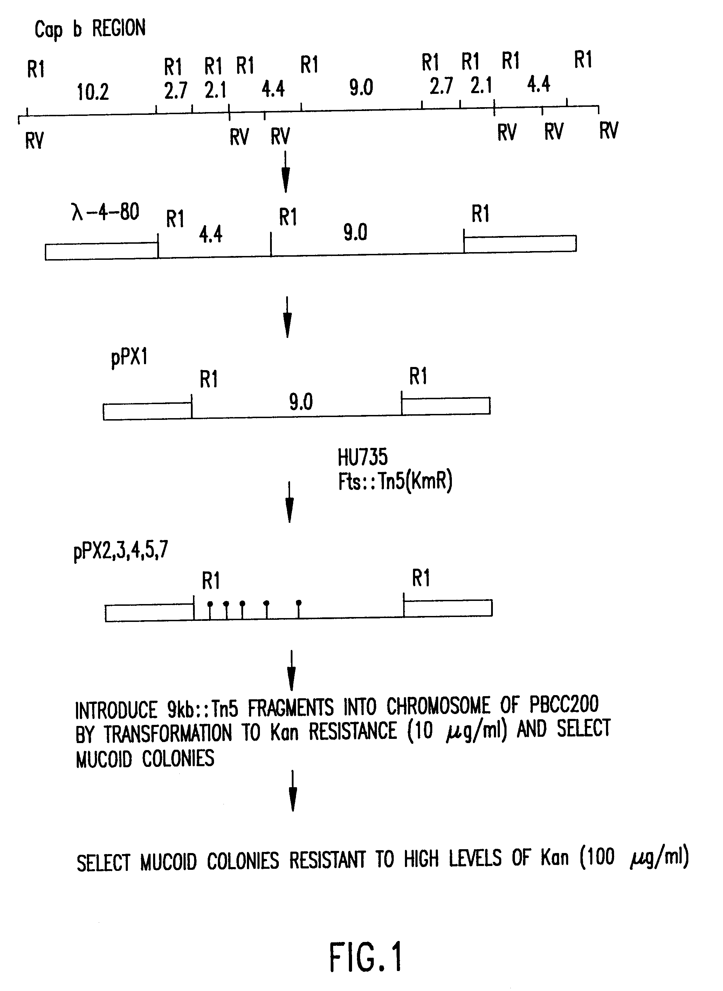

An average Hib strain, such as Eagan (PBCC200), generally produces about 100-200 µg/ml of PRP under standard culture conditions (Brain heart infusion, NAD and hemin). The Cap b region of the Hib chromosome is involved in the biosynthesis of the capsular polysaccharide, and contains a tandem duplication of an 18 Kb segment of DNA (Hoiseth et al., PNAS USA 83:1106-1110, 1986). At least a portion of both copies is necessary, since it has been demonstrated that the precise deletion of one copy of the tandem duplication results in the loss of capsule expression (Hoiseth et al., supra). Spontaneous deletion in this region occurs at a frequency of 0.1-0.3%, and forms the basis for the reported genetic instability of type b capsule expression (Pittman, M., J. Exp. Med. 53:471-492, 1931). Kroll et al. (Cell 53:347, 1988) have identified a gene (Bex A) which is essential for capsule expression and maps in an area overlapping the novel join region (Fig. 1, junction of 4.4 and 9.0 KbRI fragments) between the two copies and, thus, may partially explain the need for duplication.

It has previously been observed by Hoiseth (personal communication) that, when a 9 Kb EcoRI fragment of the Cap b region of the Hib chromosome containing a Tn5 (Km$^+$) insertion was introduced into the chromosome of strain Eagan (PBCC200) by transformation, a small percentage of the transformants resistant to 5 µg/ml of kanamycin exhibited a mucoid phenotype when grown in solid medium. Anecdotal evidence suggested that these isolates produced more PRP than the parental strains, although no characterization of the isolates was ever done, and no concrete quantification of this observation is available. No other reports of enhanced PRP production in H. influenzae have been made.

The present invention now provides a novel stable recombinant strain of H. influenzae which produces PRP in substantially higher quantities than known strains of H. influenzae, but which PRP is otherwise identical immunologically to that produced by other strains. This novel strain is useful in production of PRP which is suitable as a component in vaccine formulations.

## SUMMARY OF THE INVENTION

The present invention relates to a novel Haemophilus influenzae strain capable of producing at least twice the amount of PRP as a parental strain from which it is derived, under equivalent culture and cell density conditions. In a preferred embodiment for the novel strain is derived from Eagan strain PBCC200. Preferably the novel strain is capable of producing at least 400 µg/ml of PRP, whereas the Eagan strain PBCC200 produces about 100-200 µg/ml. In a preferred embodiment, the novel strain produces PRP having substantially identical biological characteristics as the PRP of the parental strain, particularly with regard to immunogenicity of the PRP. In addition, a method is provided for producing a microorganism capable of yielding significantly increased amounts of PRP, this method comprises the steps of culturing Haemophilus influenzae bacterium containing a selectable marker gene, such as antibiotic resistance, inserted into the gene of the Cap b locus, under conditions favoring selection for the marker, and selecting those bacteria surviving in the presence of a selection agent; repeating these steps with increasing amounts of the selection agent, whereby the surviving bacteria are capable of production of PRP at a level at least twice that of the starting bacterium.

## BRIEF DESCRIPTION OF THE FIGURES

**Figure 1.** Construction of Hib Strains Overproducing PRP. R1 and RV denote the restriction enzymes EcoR1 and EcoRV, respectively. All DNA fragment sizes are in kilobase pairs. = site of Tn5 insertion in pPX2, 3, 4, 5 and 7, respectively.

**Figure 2.** Growth of PBCC200, 181 and 197 is compared (incubation at 37°C). Growth is monitored using the Klett colorimeter (green filter).

**Figure 3.**

A. Southern blot of EcoR1 digested (lanes 1-3), or EcoRV digested (lanes 4-6) chromosomal DNA isolated from PBCC200 (lanes 1 and 4), PBCC181 (lanes 2 and 5) or PBCC197 (lanes 3 and 6) and probed with $^{32}$P-labelled 9 kb EcoR1 fragment from pPX1. Fragment sizes are estimated using λ-HindIII fragments as standards and are reported in kilobase pairs.

B. Agarose gell containing EcoR1 digested chromosomal DNA from PBCC200, PBCC181 and PBCC197 (lanes 2-4). Lane 1 contains λ-HindIII size standards. The arrows and numbers on the right denote the Cap b DNA fragments which are amplified.

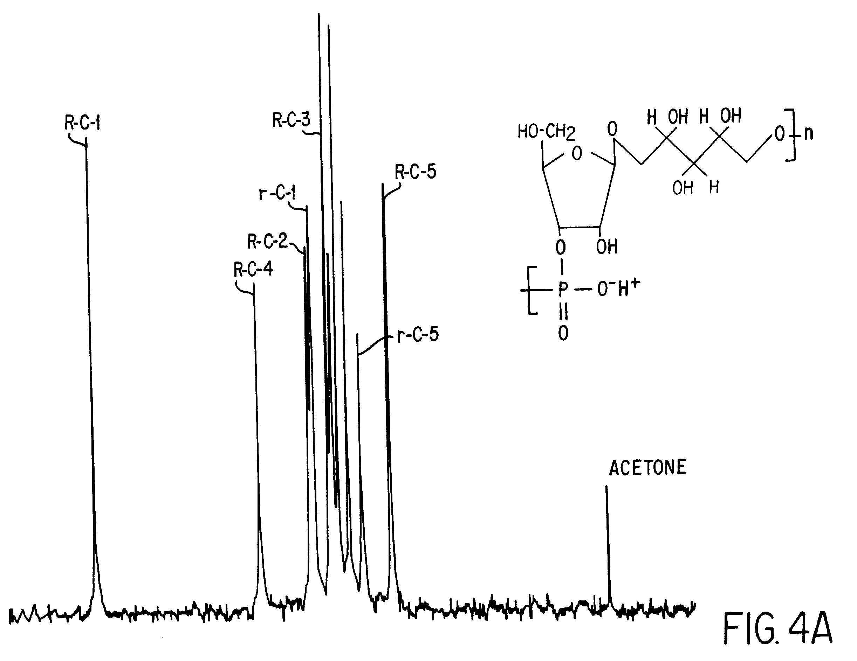

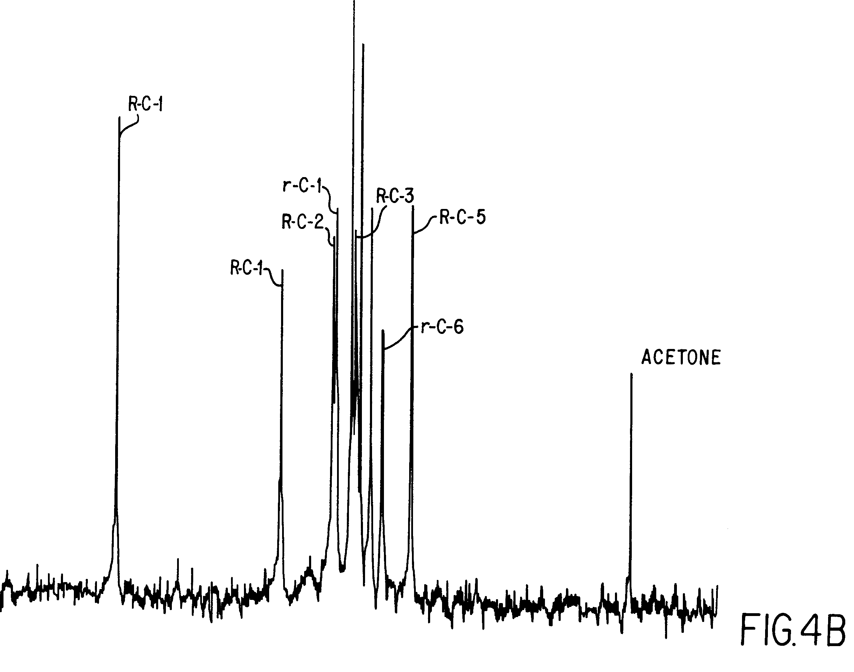

**Figure 4.** A comparison of the $^{13}$C NMR spectra of PRP produced by PBCC200 (A) and PBCC197(B). R=ribose; r=ribitol.

## DETAILED DESCRIPTION OF THE INVENTION

The present strain is constructed by insertion of a gene encoding a selectable trait into the 9 Kb EcoRI fragment of the Cap b region of the Hib chromosome, and introducing that fragment into a normal PRP-producing strain of Hib. The parental strain used in the following Examples is strain Eagan (PBCC200), but any other Hib strain can also be used for this purpose (Musser et al., Rev. Infect. Dis. 12:75, 1990).

The strain according to the Examples provided herein is prepared as follows: A recombinant phage is selected from a bank containing chromosomal Hib DNA on the basis of its ability to transform a b⁻ mutant to b⁺. Production of PRP by the transformation is confirmed by reaction with anti-PRP antiserum. Restriction endonuclease analysis of the selected phage DNA reveals the presence of both 4.4 Kb and 9 Kb EcoRI fragments. The 9 Kb fragment is purified and then cloned into an appropriate plasmid. The plasmid so constructed is transformed into a suitable host cell, such as E. coli. This host cell is then used in a mating with a second compatible strain containing the transposable element Tn5 (km$^r$), which contains a kanamycin resistance gene, or any other transposon containing a selectable marker, which transposon is compatible with the chosen? host. The mixture is then plated on selection media which selects for the transposon insertions into the plasmid. This selection results in a number of plasmids having insertions at different points in the 9 Kb fragment. The presence of the transposon insertion within the EcoR1 is verified by restriction enzyme analysis. Examples of various plasmids resulting from Tn5 insertion are summarized in Table 2.

A mixture of the isolated, restriction enzyme-digested plasmids are used to transform a strain of H. influenzae exhibiting normal production of PRP. Transformed cells are cultured on a medium containing 10 μg/ml kanamycin; although several small colonies remain after selection, only large mucoid colonies are purified for further culture. A single strain, PBCC181, is isolated as producing 2.5 times more PRP than the parental strain.

PBCC181 is used to create further derivatives which product large quantities of PRP. The 181 strain is plated onto a medium containing 100 μg/ml of kanamycin. After 48 hours of incubation, a single very mucoid colony is observed. This colony is purified and a single isolate saved for further study; this isolate is named PBCC197.

Further comparisons of the 197 strain with the 181 and 200 (parental) strains show that the growth characteristics are similar in each. Nonetheless, the two derivative strains produce significantly higher levels of PRP than the control strain. Measurements of turbidity and viable cell counts are performed in each, and it is determined that the increase is not due to increased number of cells in the derivative strains. The 197 strain is particularly productive, yielding about 3.5 times the quantity of PRP as the parental strain, or about 500 μg/ml.

The PRP of strain 197 is purified and is shown to be substantially identical to that of PBCC200. The 197 polysaccharide meets the same chemical specifications for vaccine as the parental strain. In particular, levels of contaminating nucleic acid, protein, and endotoxin, as well as size (based on KD), are similar to those of the 200 strain. NMR spectrum of the two products are superimposable, indicating structural identity (Figure 4). Immunochemical comparisons by rocket immunoelectrophoresis and ELISA indicate that the two polysaccharides are immunochemically indistinguishable. When conjugates of the PRP from 197 and 200 with CRM197 are used to immunize mice, the immunogenicity of the two conjugates is virtually identical. Thus, it appears that there is no significant difference between the PRP of strain 197 and that of strain 200.

The above description and the following examples relate specifically to the production of PBCC197. However, it will be readily understood that the practice of the invention is not limited to this strain, and similar strains can be prepared using publicly available starting materials. For example, following the procedures disclosed herein, any Hib strain may be used as the parental strain and any transposon containing a selectable marker can be used to achieve insertion of the marker into the Cap b fragment. The resulting strains should produce PRP having substantially the same biological and chemical characteristics as the parental PRP, but should produce at least twice the amount of PRP as the parental strain when cultured under identical conditions. Methods for determination of biological and/or chemical equivalence of PRP so produced are exemplified below. Preferably, the micronoganism is maintained in the presence of the appropriate selection agent.

The invention is further illustrated in the following non-limiting examples.

## EXAMPLES

### I. MATERIALS AND METHODS

The following represent basic techniques referred to in the later Examples.

### A. Media and Growth Conditions

Strains of H. influenzae are grown at 37°C in Brain Heart Infusion (BHI) medium containing 10 μg/ml of hemin and 2 μg/ml of β-nicotinamide adenine dinucleotide (NAD) (BHI-XV). E. coli strains are grown at 37°C in LB medium (Maniatis et al. Molecular Cloning: A Laboratory Manual, CSHL, 1982) which is supplemented with 100 μg/ml of ampicillin (Ap) and, or 30 μg/ml of kanamycin (Km) when growing strains containing plasmids.

### B. Preparation of DNA

A rapid small scale procedure for the isolation of bacteriophage λ DNA is performed using a plate lysate method described previously (Maniatis et al., supra).

Plasmid DNA is prepared from 1 ml cultures of E. coli using alkaline-lysis method as described by Ish-Horowitz and Burke (Nucl. Acids Res. 9:2989, 1989). A large-scale isolation procedure is performed essentially as described (Ish-Horowitz and Burke, supra) with the following changes. After precipitation of the DNA with isopropanol, the pellet is resuspended in 5 ml of 1X TE (10 mM Tris, pH 8, 1 mM EDTA) and digested with 10 μg/ml of RNase A for 20 minutes at 37°C. The mixture is extracted twice with phenol and once with chloroform-isoamyl alcohol (24:1). The sample is made 0.3 M with sodium acetate and the DNA is precipitated by adding 2.5 volumes of 95% ethanol and placing at -70°C for 15 minutes. Following centrifugation at 12,000 rpm for 30 minutes at 20°C (SS34 rotor), the pellet is resuspended in 1X TE. The DNA is further purified by centrifugation through cesium chloride gradients (Maniatis et al., supra).

Large molecular weight chromosomal DNA is isolated from H. influenzae strains using a procedure described by Moxon et al. (J. Infect. Dis. 143:517-523, 1981).

### C. Preparation of Competent Cells

High titer lysates of phage λ are prepared using E. coli as described by Maniatis et al. (supra).

### D. Preparation of Competent Cells

E. coli is made competent for transformation by plasmid DNA using calcium chloride treatment (Mendel and Hiza, J. Mol. Biol. 53:154, 1970). Competent H. influenzae are prepared by growing to early log-phase ($A_{600}$=0.1-0.15) in heart infusion broth (HI, Difco) supplemented with NAD and hemin. The cells are washed, and resuspended in 1/2 volume of Herriott's MIV medium (Herriott et al., J. Mol. Biol. 53:154, 1970) and incubated at 37°C for 100 minutes: The transformation reaction is carried out by adding 1/4 μg of transforming DNA or 50 μl of a high titer λ lysate to 1 ml of competent cells followed by incubation at 37°C for 30 minutes without shaking. The cultures are incubated, an additional hour at 37°C with gentle shaking and then a 0.1 ml aliquot is plated on the appropriate medium.

### E. Restriction Enzyme Analysis and Cloning Techniques

DNA is analyzed by digestion with restriction endonucleases using the conditions recommended by the supplier. Restricted DNA is electrophoresed in 0.7 or 1.0% agarose gels using a Tris-borate, EDTA buffer system containing 0.5 μg/ml of ethidium bromide as described by Maniatis et al. (supra). The DNA is visualized by trans-illumination with UV light. Purification of restriction fragments from low-melting temperature agarose gels is accomplished by heating the excised agarose to 70°C until completely melted, adding 2 volumes of TE followed by extraction with phenol. DNA in the aqueous phase is recovered by ethanol precipitation. The pellet is washed once with 70% ethanol and resuspended in TE. DNA ligations are performed as described (Maniatis et al., supra).

## F. Southern Transfer and Hybridization

Transfer of DNA fragments to nitrocellulose is done as described by Smith and Summers (Anal. Biochem. 109:123-129, 1980). The filters are prehybridized, hybridized and washed as described (Maniatis et al., supra). $^{32}$-P-labelled probes are prepared using a nick-translation kit (BRL) and [$\alpha$-$^{32}$-P]dATP (Amersham).

## G. Purification of PRP

A micropurification procedure is used to estimate the amount of PRP present in culture supernatants. One ml of culture is transferred to a 1.5 ml microfuge tube and the cells are removed by centrifugation for 10 minutes at 10,000 x g. Four-hundred $\mu$l of the culture supernatant are transferred to a clean microfuge tube and 200 $\mu$l of 0.5 M hexadecyltrimethylammonium bromide (HB) are added. The sample is rocked at room temperature for 1 hour and then centrifuged for 10 minutes as above. The pellet is resuspended in 0.2 ml of .04 M NaCl and 0.6 ml of 95% ethanol is added and the sample is rocked at room temperature for 1 hour. The PRP is harvested by centrifugation for 10 minutes and then resuspended in 0.4 ml of dH$_2$O.

The PRP concentration is determined by the orcinol pentose assay (Ashnell, G., Meth. Enzymol. 31:73, 1957).

## II. RESULTS

## A. Isolation of Recombinant Phage Containing Cap b DNA

A recombinant phage ($\lambda$4-80) was isolated from a bank containing chromosomal DNA from H. influenzae strain KW20b (Moxon and Vaughn, J. Infect. 143:517-523, 1981) as follows: the construction of this library has been described previously (Moxon et al., J. Clin. Invest. 73:298-306, 1984). The library (5 x 10$^6$ pfu/ml) is diluted to yield 10 pools containing approximately 10$^3$ phage per 10 $\mu$l. Each pool is amplified using strain K802 and tested for the ability to transform strain sec-1 to b$^+$. Transformants are identified by their iridescent phenotype. Two of the ten pools are able to carry out this transformation. One pool is diluted and plated for single plaques. Subpools containing 10 plaques each are amplified and tested for the b$^+$ transforming DNA as before. Eventually, a single plaque is identified based on the ability to transform a b$^-$ mutant strain of Hi (Sec-1) to b$^+$ with the phage DNA; the presence of colonies producing the type b capsule is determined by visual inspection of BHI-XV plates containing 500-1000 cfu each for the presence of colonies exhibiting the iridescent phenotype characteristic of type b organisms. This isolate which yields phage containing Cap b DNA is identified as $\lambda$4-80 (Figure 1).

Further proof that the transformants are producing PRP is obtained by rocket immunoelectrophoresis using anti-PRP antiserum. The sec-1 strain contains a mutation within the 4.4 kb EcoRI fragment of the Cap b locus (Ely et al., J. Bacteriol. 167:44-48, 1986) (See Figure 1). Therefore, it is expected that $\lambda$4-80 contains at least a portion of the 4.4 kb fragment necessary to correct this mutation upon transformation. Restriction endonuclease analysis of the DNA from $\lambda$4-80 confirms the presence of the intact 4.4 kb fragment and shows that the 9 kb fragment, adjacent to the 4.4 kb fragment in the wild type cap b region is present as well. The 9 kb fragment is purified and cloned into the EcoRI site of pBR322 to form pPX1 (Figure 1). Five derivatives of pPX1 containing the transposable element TN5 (Km$^r$) in different locations within the 9 kb fragment are constructed as follows and listed in Table 2 (pPX2, 3, 4, 5, and 7): TN5 insertions into pPX are constructed by diluting overnight cultures of HB101(pPX1) and HU735 into 10 ml of LB. Each culture is grown (HU735 at 32°C) until cell densities reach mid-log phase (A$_{500}$=0.4). One ml samples of each culture are mixed and incubated at 32°C for 1 hour without shaking and then for 2 hours with gentle rotary shaking. The mating mixture is diluted 1:20 into LB containing 100 $\mu$g/ml of ampicillin and 300 $\mu$g/ml of kanamycin followed by incubation overnight at 42°C. These conditions select for TN5 insertions into the multicopy plasmid and loss of the F'$_{ts}$ element containing a mutation which inhibits replication 42°C. Plasmid DNA is prepared from the overnight culture and used to transform HB101 to Ap and Km resistance.

## TABLE 1

| LIST OF STRAINS | | |
|---|---|---|
| Strain | Genotype/Comments | Source/Reference |
| E. coli | | |
| HB101 | hsdS20(r⁻, m⁻), recA13 ara-14, proA2, lacY1, galK2, rps120(Str^r), xyl-5, mtl-1, supE44 | Boyer and Roulland-Dussoix, J. Mol. Biol. 41:459, 1969 |
| Hu735 | thi-1, thr-1, leuB6, lacY1 tonA2, supE44, trpE5, recA56 F'(ts, lac⁺, trp⁺, zzff28::TN5) | (R. Hull) |
| K802 | hsdR⁺, hsdM⁻, gal⁻, met⁻, supE | Wood, J. Mol. Biol. 16:118, 1966 |
| H. influenzae | | |
| PBCC200 | type b, CSF isolate (Eagan) | Andersen et al., J. Clin. Invest. 51:298-306, 1984 |
| sec-1 | unencapsulated type b mutant | Moxon et al., J. Clin. Invest. 73:298-306, 1984 |
| PBCC181 | mucoid, resistant to 10 µg/ml Km | This Study |
| PBCC197 | mucoid, resistant to 100 µg/ml Km | This Study |

## TABLE 2

| LIST OF PLASMIDS | | |
|---|---|---|
| Plasmid | Genotype/Comment | Source/Reference |
| pBR322 | bla⁺, tet⁺ | Bolivar et al., Gene 2:95, 1977 |
| pPX1 | contains 9 kb R1 Cap b fragment from λ4-80 | This Study |
| pPX2 | bla⁺, apt⁺, pPX1::TN5-1 | This Study |
| pPX3 | bla⁺, apt⁺, pPX1::TN5-2 | This Study |
| pPX4 | bla⁺, apt⁺, pPX1::TN5-3 | This Study |
| pPX5 | bla⁺, apt⁺, pPX1::TN5-4 | This Study |
| pPX7 | bla⁺, apt⁺, pPX1::TN5-8 | This Study |
| pPX12 | bla⁺, apt⁺, tet⁺, pBR322::TN5 | This Study |
| pPX13 | bla⁺, apt⁺, 9kb EcoRI fragment from PBCC197 | This Study |

## B. Isolation of PRP-Overproducing Strain PBCC181 and PBCC197

Strain PBCC181 is constructed by transforming competent Hib strain PBCC200 with a mixture containing PvuI digested pPX2, 3, 4, 5, and 7 DNA (Table 2). The transformed cells are plated onto BHI-XV media containing 10 - ug/ml of kanamycin and incubated at 37°C. After two days numerous small colonies (<1 mm) appears. However, two larger mucoid colonies are present. These are purified on BHI-XV Km₁₀ medium. After overnight incubation, one of these isolates is very mucoid. A single colony isolate designated PBCC181 is picked for further characterization. This strain requires both hemin and NAD for growth and reacts with type b-specific antiserum (but not with type a, c, d, e or f). Strain PBCC181 produces 2.5 times more PRP than the parental strain PBCC200, suggesting that the mucoid phenotype is indicative of PRP overproduction.

The mechanism by which PRP synthesis by PBCC181 is enhanced is not clear. The two alternate explanations are plausible: either the Tn5 insertion interrupts a gene that has a negative effect on capsule production or that the portion of the Cap b gene surrounding the Tn5 insertion undergoes a genetic amplification event triggered by selection on kanamycin. If the latter is true, it should be possible to isolate derivatives of PBCC181 containing additional copies of the Tn5 and adjacent Cap b sequence by plating onto medium containing concentrations of kanamycin >10 ug/ml. To test this, an overnight culture of PBCC181 is plated onto BHI-XV containing 100 ug/ml of kanamycin. After 48 hours of incubation a single, very mucoid colony is observed. This colony is purified and a single colony isolate is saved for further study (PBCC197). This isolate also requires X and D factors (Hemin and NAD) and reacts with only type b-specific antiserum.

### C. Growth and PRP Production

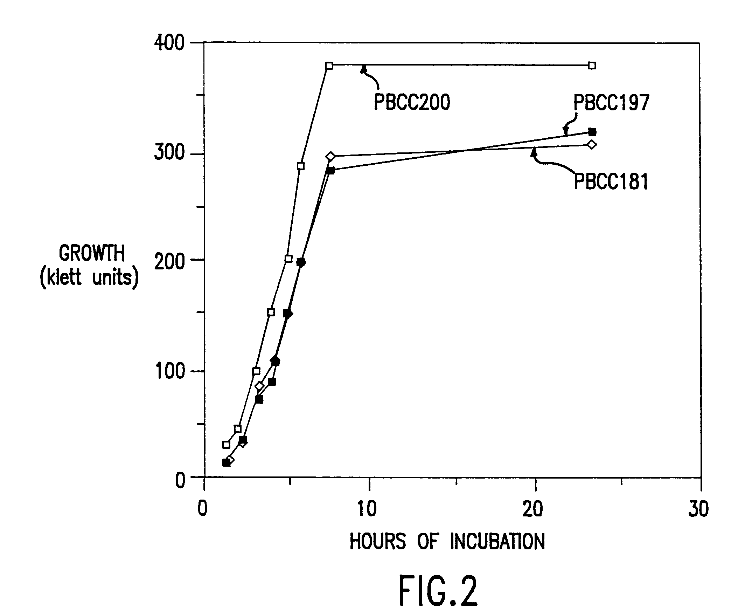

Growth and PRP production by strains PBCC200, 181 and 197 are compared. Cultures are incubated at 37°C. Growth is monitored using the Klett colorimeter (green filter) (Figure 2) and PRP production is assayed after 23 hours of incubation. PRP production by these strains is shown in Table 3. The growth characteristics of the overproducing strains are very similar to PBCC200 yet they produce 2.5 (PBCC181) and 3.5 (PBCC197) times more PRP than the control strain. The measurements of turbidity and viable cell counts indicate that the additional PRP is not due to an increased number of cells, but to an enhanced synthetic capacity. Based on these results, PBCC197 is chosen for further evaluation.

### TABLE 3

### PRP PRODUCTION BY PBCC181, 197 AND 200

| Strain | EXPT | PRP($\mu$g/ml)* | AVG. PRP | FOLD INCREASE |
|---|---|---|---|---|
| PBCC200 | 1 | 126 | | |
| | 2 | 183 | | |
| | 3 | 172 | | |
| | 4 | 160 | 160 | - - - |
| PBCC181 | 1 | 315 | | |
| | 2 | 412 | | |
| | 3 | 408 | | |
| | 4 | 434 | 392 | 2.5 |
| PBCC197 | 1 | 571 | | |
| | 2 | 549 | | |
| | 3 | 588 | | |
| | 4 | 560 | 567 | 3.5 |

Notes:

* Each PRP value is based on the average of triplicate pentose assays from PRP samples isolated from duplicate cultures.

@ Fold increase over PBCC200

### D. Genetic Organization of the Cap b Locus of PBCC181 and PBCC197

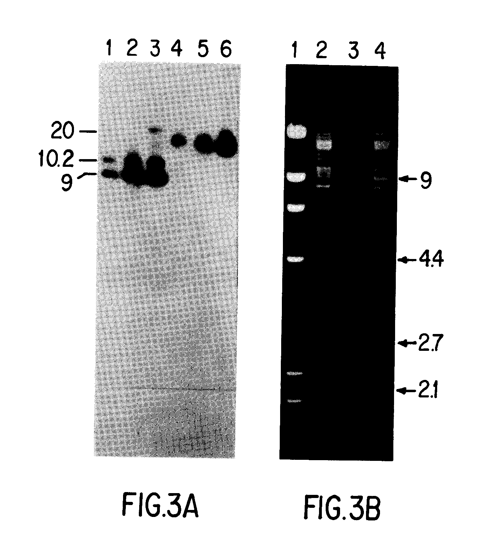

The genetic basis for the overproduction of PRP by these strains is investigated. If the mechanism is due to a genetic amplification event, then Southern hybridization experiments using chromosomal DNA from those strains should show an increase in the copy number of the 9 Kb EcoRI Cap b fragment when the blots are hybridized with the homologous probe. Equal amounts of chromosomal DNA from PBCC200, 181 and 197 is digested with EcoRI or EcoRV, electrophoresed in 0.7% agarose and transferred to nitrocellulose. The blot is probed with the $^{32}$P-labelled 9 Kb EcoRI fragment from pPX1. The results are shown in Figure 3. As expected, the probe hybridizes to 9, 10.2 and 20 Kb EcoRI fragments and to an 18 Kb EcoRV doublet from strain PBCC200 (Hoiseth et al., supra). The probe hybridizes to fragments of these sizes from PBCC181 and PBCC197 also. However, the level of hybridization to the 9 Kb fragment from the PRP overproducing strains is much greater. The highest level of hybridization is seen with DNA from PBCC197. These data suggest that an amplification of the Cap b region occurs in the overproducing strains and that the degree of amplification correlates with the level of resistance to kanamycin and PRP production. This amplification can also be seen by visualizing the DNA fragments in the agarose gel after staining with ethidium bromide (Figure 3B). In fact, these data indicate that in addition to the 9 Kb fragment, the 2.1, 2.7 and 4.4 EcoRI fragments of the Cab b repeat region of PBCC197 are amplified as well (Figure 3B, see arrows). Thus, the entire 18 Kb repeat of the Cap b locus is amplified in PBCC197. It appears that amplification of the Cap b region forms the genetic basis for PRP overproduction.

## G. Characterization of PRP Isolated from PBCC197

Purified PRP from PBCC197 is compared chemically and immunologically with purified PRP from PBCC300 to determine the extent of their similarity.

### 1. Chemical Analysis

Two samples of PRP are purified by small and large scale methods (supra) separately. Samples are submitted to the Quality Control Laboratory for analysis. A battery of tests used to evaluate other PRP vaccines is used. The results are shown in Table 4. Both batches of PRP isolated from PBCC197 meet the chemical specifications required for vaccine use. The levels of contaminating nucleic acid, protein and endotoxin are similar to those found in samples of PRP from strain PBCC200. In addition, the sizes (based on Kd) of PRP from strain PBCC200 is similar to PRP purified from strain PBCC197.

TABLE 4

| QUALITY CONTROL TESTING OF PRP FROM PBCC197 | | | |
|---|---|---|---|
| | | PRP BATCH | |
| TEST | SPECIFICATIONS | LARGE SCALE | SMALL SCALE |
| Pentose | >1 mg/ml | 5.96 mg/ml | 3.84 mg/ml |
| Phosphate | >1 mg/ml | 5.97 mg/ml | 3.56 mg/ml |
| Nucleic Acid | <0.8% | 0.28% | 0.54% |
| Protein | <0.8% | 0.29% | 0.29% |
| Kd | <0.6 | 0.473 | 0.466 |
| Endotoxin | <0.2 E$\mu$/Ug | 0.01 E$\mu$/Ug | 0.02 E$\mu$/Ug |

### 2. $^{13}$C NMR Comparison

The $^{13}$C nuclear magnetic resonance spectra of PRP isolated from PBCC197 and PBCC200 are determined using a Varian XL-100-15 spectrometer. The spectra (Figure 4) are superimposable, thus providing evidence that the two polysaccharides are structurally identical.

### 3. Immunochemical Comparison

Immunoelectrophoresis:

The immunochemical properties of PRP isolated from PBCC197 and PBCC200 are compared using immunoelectrophoresis as described in the methods section. The Kd values for the two polysaccharides are 0.466 and 0.5 respectively. Anti-PRP polyclonal antiserum raised in burro is used.

The PRP samples are compared using standard rocket immunoelectrophoresis. A 1%(w/v) agarose gel is cast, containing 1%(v/v) antiserum. Samples containing 8.75, 17.5, 35 and 70 ng of PRP are electrophoresed at 70 V/cm for 30 minutes using 10 mM Tris barbiturate buffer (pH 8.6) followed by silver staining. If the PRP produced by PBCC197 and 200 is identical with respect to recognition by polyclonal antibodies, then the pattern and height of the rockets for corresponding amounts of PRP should be identical. Experimental results show the rockets for PRP PBCC197 are identical in pattern, but slightly higher than those observed for PRP from PBCC200: 39.6 cm compared to 34.5 cm. Since rocket height is directly proportional to the amount of antigen-antibody complex formed using this method, the PRP from PBCC197 is recognized as antigen by at least as many antibodies (polyclonal) as is PRP from PBCC200.

### 4. ELISA Studies:

The PRP from PBCC197 and PBCC200 are also compared for the ability to compete for binding to various anti-PRP antibodies by ELISA. Polystyrene 96 well ELISA plates (NUNC) are coated with 1 ug/ml of tyraminated PRP (PBCC200) in pre-determined concentration of antibody and varying concentrations of PRP sample and the plates are incubated for 18 hours in a humidified 37°C incubator. Anti-PRP antibodies are titrated initially on these plates to determine the optimal concentration of antibody which would give an OD of 0.5 to 0.6. Bound antibodies are detected with alkaline phosphatase-coupled goat anti-mouse antibodies.

The data in Table 5 demonstrate that PBCC197 PRP competes as well as PBCC200 PRP for binding of native PRP (PBCC200) to murine monoclonal and polyclonal anti-PRP antibodies and human polyclonal anti-PRP antibodies over a wide range of antigen concentration. This data is used to determine the concentration of the two antigens needed to inhibit 50% of binding of the native antigen to the antibodies (Table 6). These results provide further support for the claim that the PRP from PBCC197 is immunochemically indistinguishable from that produced by the strain PBCC200.

**TABLE 5**

**COMPARISON OF PRP FROM PBCC200 AND PBCC197**

**BY ANTI-PRP COMPETITION ELISA**

PERCENT INHIBITION

| Ag Conc.[a] | MAB E-117-5[b] | | Murine Poly[b] | | Human Poly(BOB)[b] | |
|---|---|---|---|---|---|---|
| | 200 | 197 | 200 | 197 | 200 | 197 |
| 10 | 99 | 99 | 99 | 99 | 94 | 96 |
| 5 | 98 | 98 | 99 | 99 | 94 | 96 |
| 2.5 | 97 | 97 | 99 | 99 | 88 | 93 |
| 1.25 | 93 | 93 | 97 | 96 | 84 | 82 |
| 0.625 | 88 | 88 | 94 | 91 | 81 | 78 |
| 0.313 | 78 | 77 | 85 | 84 | 67 | 66 |
| 0.156 | 64 | 62 | 76 | 62 | 65 | 65 |
| 0.078 | 48 | 47 | 65 | 60 | 58 | 59 |
| 0.039 | 31 | 28 | 55 | 53 | 46 | 52 |
| 0.020 | 24 | 23 | 46 | 38 | 44 | 39 |

Notes:

[a] Ag is PRP from PBCC200 or PBCC197. Amount of antigen is expressed as ug/ml.

[b] Each well received approximately 1.9 ng/ml of murine mab E117-5, 150 ng/ml of murine polyclonal antibody, or 4.4 ng/ml of human anti-PRP (BOB) antibodies as estimated by Farr assay.

**TABLE 6**

| COMPARISON OF POLYSACCHARIDES FROM PBCC200 AND PBCC197 TO ABSORB MURINE OR HUMAN ANTI-PRP ANTIBODIES | | | |
|---|---|---|---|
| Conc. of Ag Needed for 50% Inhibition (ng/ml) | | | |
| PRP | Murine mab | Murine polyclonal | Human polyclonal |
| 200 | 78 | 29 | 58 |
| 197 | 78 | 29 | 39 |

5. Immunogenicity Studies:

To evaluate immunogenicity, the PRP from PBCC197 is used to prepare oligosaccharides which are conjugated to CRM197 by reductive animation (U.S. Patent No. 4,902,506). The immunogenicity of this vaccine (HbOC-197) is evaluated by injecting 1 ug of the HbOC vaccine into 10 eight-week-old Swiss Webster mice at weeks 0 and 4. Serum samples are obtained at weeks 0, 2, 3, 4, and 5. The results shown in Table 8 compare the results obtained with HbOC-197 with the results of a similar experiment using a standard HbOC preparation (PRP from PBCC200). PRP from 197 is as immunogenic as that from PBCC200 in this assay.

**TABLE 8**

**MOUSE IMMUNOGENICITY ASSAY**

| Vaccine | Antibody Titer at Week (ug/ml) | | | | |
|---|---|---|---|---|---|
| | 0 | 2 | 3 | 4 | 5 |
| HbOC-197 | 0.1 | 2.03 | 4.33 | 4.62 | 21.66 |
| HbOC-200 | <0.1 | 3.08 | 4.51 | 4.47 | 22.58 |

Note:

Anti-PRP titers determined by RIA with OBRR Std. = 70 ug/ml

The following biological materials have been deposited with the American Type Culture Collection, 12301 Parklawn Drive, Rockville, Maryland, on December 4, 1991:

| Strain | Accession No. |
|---|---|
| Haemophilus influenzae b | |
| strain Eagan | ATCC 55257 |

**Claims**

1. A Haemophilus influenzae strain capable of producing at least 400 µg/ml of polyribosyl-ribitol-phosphate (PRP) in culture.

2. The strain of Claim 1 which has substantially the same biological characteristics as Strain PBCC197.

3. A Haemophilus influenzae strain which produces at least two times the amount of PRP as strain Eagan when cultured under identical conditions.

4. The strain of Claim 3 which produces at least three times the amount of PRP as strain Eagan when cultured under identical conditions.

5. The strain of Claim 4 which has substantially the same biological characteristics as strain PBCC197.

6. A method for producing a Haemophilus influenzae strain having the ability to produce large quantities of PRP comprising inserting into the Cap b chromosome of a parental H. influenzae strain producing normal amounts of PRP, a selectable marker, culturing the strain into which the marker is inserted in the presence of a normally inhibitory amount of a selection agent, selecting a first set of surviving microorganisms, and culturing them in the presence of higher amounts of a selection agent, and selecting a second set of the surviving microorganisms, whereby the surviving micro-organisms after at least two selection steps, are capable of producing at least twice the amount of PRP as the parental strain.

7. A Haemophilus influenzae strain produced by the method of Claim 6.

8. A recombinantly produced H. influenzae strain derived from a parental strain, wherein the recombinantly produced strain produces at least twice the amount of PRP produced by the parental strain when both strains are cultured under identical conditions.

9. The strain of Claim 8 which produces at least twice the mount of PRP as the parental strain.

10. The strain of Claim 8 which comprises a selectable marker inserted wtihin the Cap b region of Hib chromosome.

Cap b REGION

FIG.1

FIG.2

EP 0 546 349 A2

FIG.3A

FIG.3B

FIG. 4A

FIG.4B